# EUROPEAN PATENT APPLICATION

(11) **EP 4 289 414 A1**
(43) Date of publication of application: **13.12.2023**
(21) Application number: 22177956.4
(22) Date of filing: 08.06.2022
(51) Int. Cl.: A61K 9/00, A61L 27/00, C08F 220/60, C08L 33/26

(54) **TISSUE PROTECTIVE HYDROGEL**

(71) Applicant: ETH Zürich, 8092 Zürich (CH)
(72) Inventor: The designation of the inventor has not yet been filed
(74) Representative: Schulz Junghans Patentanwälte PartGmbB

(57) **Abstract**

The present invention relates to the medical use of a zwitterionic antifouling hydrogel. The hydrogel may be used in prevention of and protection against inflammation of tissue or in stabilization of a tissue implant.

## Description

The present invention relates to the medical use of a zwitterionic antifouling hydrogel. The hydrogel may be used in prevention of and protection against inflammation of tissue or in stabilization of a tissue implant.

### Background of the Invention

Inflammation, for example, in chronic diseases such as osteoarthritis or resulting from the body's immune response to implanted soft tissues, is a main cause of tissue degradation and implant failure. Due to the complex and multi-factorial nature of the inflammation, chemical inhibitors and mediators of inflammation are not very effective in controlling its detrimental effects on tissue loss.

This phenomenon is of great importance in diseases such as osteoarthritis, where there is no disease modifying drug, and the persistent inflammation continuously causes more tissue degradation.

Moreover, implantation of artificial organs or biomaterials can result in initiation of an inflammatory response. This inflammation comprises acute inflammation, chronic inflammation and foreign body reaction with multiple immune cells and cytokines involved. Through a complicated cascade of events, this inflammatory response may result in implant isolation, and in case of soft tissue implants, its degradation.

Better solutions for controlling inflammation and protecting healthy native tissues or engineered soft tissues in inflammatory environments are desirable. Thus, an effective method that can protect tissues in inflammatory environment and inhibit progressive tissue loss is of great importance and can significantly improve patient wellbeing in chronic inflammatory diseases as well as enhance performance of implant soft tissues.

Based on the above-mentioned state of the art, the objective of the present invention is to provide means and methods to provide a tissue-protecting texture preventing inflammation and tissue loss of native and implanted tissue. This objective is attained by the subject-matter of the independent claims of the present specification, with further advantageous embodiments described in the dependent claims, examples, figures and general description of this specification.

### Summary of the Invention

A first aspect of the invention relates to a pharmaceutical composition comprising:
- a linear polymer comprising, or essentially consisting of:
   i. a biocompatible zwitterionic first monomer; and
   ii. a biocompatible crosslinkable second monomer.

A particular aspect of the invention relates to the use of the pharmaceutical composition in medicine.

A second aspect of the invention relates to a pharmaceutical composition as described in the first aspect for use in prevention of and protection against inflammation of tissue.

A third aspect of the invention relates to a pharmaceutical composition as described in the first aspect for use in stabilization of a tissue implant.

In another embodiment, the present invention relates a pharmaceutical composition comprising at least one of the compounds of the present invention or a pharmaceutically acceptable salt thereof and at least one pharmaceutically acceptable carrier, diluent or excipient.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open-ended in that an item or items following any one of these words is not meant to be an exhaustive listing of such item or items or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *zwitterionic* in the context of the present specification relates to a moiety comprising, under physiological conditions, a positively charged moiety and a negatively charged moiety.

The term *backbone moiety derived from a polymerizable moiety* in the context of the present specification relates to a moiety which in monomeric form has a reactive chemical entity being used for polymerization. The backbone moiety designates the previously reactive chemical entity after the polymerization step.

The term *polymerized form* of in the context of the present specification also relates to the previously reactive chemical entity after the polymerization step. For example, acrylate in its polymerized form is polyacrylate.

The term *quaternary ammonium* in the context of the present specification relates to a positively charged nitrogen with four valences. Depending on the position within the whole molecule, a quaternary ammonium may be, for example, -N(CH₃)₃⁺, -N(CH₃)₂⁺-, -NR₃⁺, or -NR₂⁺- with R being C₁-C₄ alkyl.

The term *tertiary ammonium* in the context of the present specification relates to a positively charged nitrogen with one hydrogen, for example, -NH(CH₃)₂⁺,-NHR⁺-, -NHR₂⁺, or -NH(CH₃)⁺- with R being C₁-C₄ alkyl.

The term *secondary ammonium* in the context of the present specification relates to a positively charged nitrogen with two hydrogens, for example, -NH₂(CH₃)⁺, -NH₂R⁺, or -NH₂⁺-with R being C₁-C₄ alkyl.

The term *primary ammonium* in the context of the present specification relates to a positively charged nitrogen with three hydrogens, which is -NH₃⁺.

The term ECM in the context of the present specification relates to extracellular matrix.

As used herein, the term *pharmaceutical composition* refers to a compound of the invention, or a pharmaceutically acceptable salt thereof, together with at least one pharmaceutically acceptable carrier.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

As used herein, the term *treating* or *treatment* of any disease or disorder (e.g. inflammation) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

### Detailed Description of the Invention

A first aspect of the invention relates to a pharmaceutical composition comprising:
- a linear polymer comprising, or essentially consisting of, monomeric units selected from:
   i. a biocompatible zwitterionic first monomer; and
   ii. a biocompatible crosslinkable second monomer;
particularly for use in medicine.

An alternative of the first aspect of the invention relates to a pharmaceutical composition comprising:
- a linear polymer comprising, or essentially consisting of, monomeric units selected from:
   i. a biocompatible zwitterionic first repeating unit; and
   ii. a biocompatible crosslinkable second repeating unit;
particularly for use in medicine.

The first and second repeating unit will be described as first monomer and second monomer, respectively, hereafter.

In certain embodiments, the first monomer comprises
a. a positively charged moiety;
b. a negatively charged moiety; and
c. a backbone moiety derived from a polymerizable moiety.

In certain embodiments, the polymerizable moiety is selected from the group comprising acryl, methacryl, vinyl.

In certain embodiments, the first monomer is described by a general formula (I) or (II): 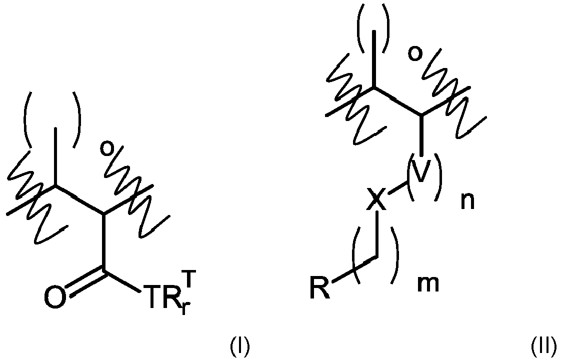 wherein
- T is a heteroatom moiety comprising nitrogen or oxygen;
- r is 1 if T is NH or O, and r is 2 if T is N;
- R^{T} is 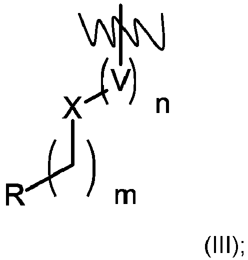
- one of X and R is a positively charged moiety, and the other one is a negatively charged moiety;
- V is selected from the group of -CH₂-, -OCH₂CH₂-, or -CH₂CH₂O-;
- n is selected from 0, 1, 2, 3, 4, 5, 6, 7, 8, 9, 10;
- m is selected from 0, 1, 2, 3, 4;
- o is selected from 0 and 1.

Positively and negatively charged moieties are selected from the following table:

| Moiety | X | R |
|---|---|---|
| positively charged | quaternary ammonium tertiary ammonium secondary ammonium | guanidine quaternary ammonium tertiary ammonium secondary ammonium primary ammonium |
| negatively charged | phosphate thiophosphate | carboxyl sulfate |

In other words, the first monomer (I) is a derivative of acrylic acid or methacrylic acid, or the first monomer (II) is a derivative of polyvinyl alcohol or a higher alkyl homologue.

Formula (I) may also be described as:

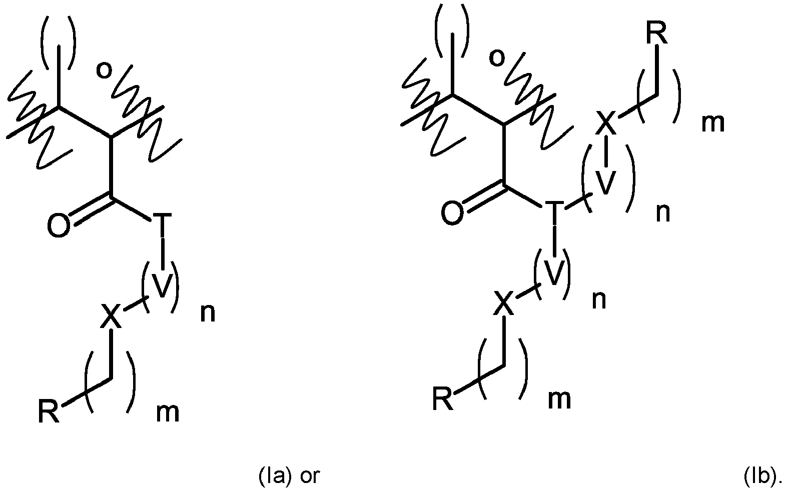

In certain embodiments, the positively charged X is a quaternary ammonium. In certain embodiments, the positively charged R is selected from guanidine, a quaternary ammonium, a primary ammonium.

In certain embodiments, the first monomer is described by a general formula (I).

In certain embodiments, T is selected from O, N, NH. In certain embodiments, T is NH.

In certain embodiments, V is -CH₂-.

In certain embodiments, n is selected from 1, 2, 3.

In certain embodiments, m is selected from 0, 1, 2.

In certain embodiments, the first monomer comprises a carboxybetaine moiety, a sulfobetaine moiety, a sulfabetaine moiety or a phosphorylcholine moiety. In certain embodiments, the first monomer is selected from a polymerized form of CBAA (carboxybetaine acrylamide), CBMA (carboxybetaine methacrylate), SBMA (sulfobetaine methacrylate), MPC (2-methacryloyloxyethyl phosphorylcholine). In certain embodiments, the first monomer is a polymerized form of CBAA.

In certain embodiments, the pharmaceutical composition additionally comprises a first crosslinking agent capable of crosslinking said second monomer.

In certain embodiments, the first crosslinking agent is an enzyme.

In certain embodiments, the composition additionally comprises a substrate for said crosslinking agent.

In certain embodiments, the enzyme is horse-radish peroxidase, and the substrate is H₂O₂.

In certain embodiments, the enzyme is Factor XIII.

In certain embodiments, the polymer comprises a crosslinkable moiety, wherein crosslinking of the crosslinkable moiety is induced by heat or by light.

Crosslinking via light or heat can be performed using light- or heat- sensitive initiators and a radical donor comonomer.

In certain embodiments, the second monomer is described by a general formula (IV): 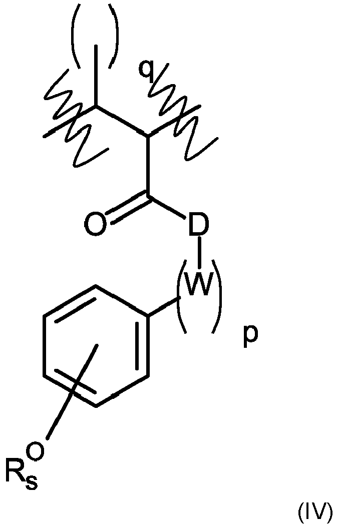 wherein
- p is selected from 0, 1, 2, 3, 4, 5, 6, 7, 8, 9, 10;
- q is selected from 0 and 1;
- D is a heteroatom moiety;
- W is selected from the group of -CH₂-, -OCH₂CH₂-, or -CH₂CH₂O-;
- R^{O} is OH;
- s is selected from 1, 2.

In certain embodiments, p is selected from 1, 2, 3.

In certain embodiments, s is 1.

In certain embodiments, W is -CH₂-.

In certain embodiments, D is selected from O, NH.

In certain embodiments, the second monomer comprises a crosslinkable moiety selected from tyramine, phenol, catechol, resorcinol.

In certain embodiments, the second monomer is selected from a polymerized form of tyramine acrylamide, phenol acrylamide, catechol acrylamide, resorcinol acrylamide. In certain embodiments, the second monomer is a polymerized form of tyramine acrylamide. The OH group at the aromatic ring will radicalize upon exposure to crosslinkers and form a covalent crosslinking bond between polymer chains.

In certain embodiments, a ratio of the first monomer to the second monomer is 20:1 to 5:1. In certain embodiments, a ratio of the first monomer to the second monomer is ∼10:1. The ratio affects the degree of crosslinking of the hydrogel.

In certain embodiments, the polymer has a molecular mass of 50 kDa to 1000 kDa. In certain embodiments, the polymer has a molecular mass of 100 kDa to 500 kDa. In certain embodiments, the polymer has a molecular mass of -200 kDa.

A second aspect of the invention relates to a pharmaceutical composition as described herein for use in prevention of and protection against inflammation of tissue.

Cartilage tissue affected with osteoarthritis (OA) in different joints: knee, hip, hand, spine, etc., is one important target. Also, other types of arthritis could benefit i.e., rheumatoid arthritis (RA), inflammatory arthritis, and ankylosing spondylitis. Moreover, other musculoskeletal (MSK) conditions including tendinopathy, degenerative disc disease and bursitis are other examples which could benefit from this formulation. Outside of the MSK, dry eye disease, endometriosis, periodontitis, leaky gut syndrome and in general any condition in which there is inflammatory component are a potential target.

In certain embodiments, the pharmaceutical composition is formulated for *in-situ* administration. The formulation can be injected with a double syringe keeping two crosslinkers separately until injection in situ, or via two successive injections wherein the first injection includes the polymer plus the crosslinking agent and the second injection provides the substrate. The latter method also provides time for penetration of polymer chains into the tissue. The pharmaceutical composition is an injectable formulation that will be injected locally to the location of interest, such as the joint.

In certain embodiments, the composition is administered into an inflamed tissue, or the pharmaceutical composition is administered in order to protect the tissue from inflammation.

In certain embodiments, the inflammation of tissue results from an inflammatory disease. In certain embodiments, the inflammation of tissue results from osteoarthritis, or rheumatoid arthritis.

In certain embodiments, the tissue is cartilage tissue.

A third aspect of the invention relates to a pharmaceutical composition as described herein for use in stabilization of a tissue implant. The coating is supposed to be applied on the implant before implantation and before the implant is going to be in contact with the host tissue.

An alternative of the third aspect of the invention relates to a pharmaceutical composition as described herein for use in medicine by administration as a coating to a tissue implant.

One main target of this coating is any implant made of ECM-like tissue which is produced in vitro. This means that there has been a starting scaffold encapsulating the cells and the cells have produced their own ECM over time resulting in matured tissue. In certain embodiments, the starting scaffold to produce the tissue is selected from the group comprising hydrogel, lyophilized scaffold, electrospun scaffold. Hydrogels are the most widely used scaffolds in tissue engineering.

In certain embodiments, the implant is made of decellularized allografts or xenografts which suffer from immune rejection in vivo.

The coating can be applied to acellular scaffolds if the chemistry of the polymer used for scaffold fabrication is compatible with the chemistry used for the crosslinking of the coating. This conditioning is because in case of ECM-based implants, either in vitro cultured or decellularized, the coating is crosslinked to the native tissue, but if there is no ECM, the coating should be crosslinked to the acellular scaffold itself.

In certain embodiments, the pharmaceutical composition is administered as a crosslinkable protective penetrant for engineered tissues before implantation. In certain embodiments, the engineered tissue is a soft tissue.

In certain embodiments, the tissue implant is a hydrogel, a cell-based scaffold or an ECM-like scaffold.

In certain embodiments, the tissue implant is a cartilage (articular or auricular) implant. In certain embodiments, the tissue implant is a heart valve prosthesis. In certain embodiments, the tissue implant is a vasculature graft. In general, whenever a tissue implant will be in contact with host tissue, it is prone to immune rejection and degradation, and here the coating of the invention can be useful to prevent the immune rejection and help in implant survival in the initial phases of acute inflammatory response.

In certain embodiments, the pharmaceutical composition is capable of penetrating into tissue, and the pharmaceutical composition is crosslinkable to extra-cellular matrix of cells. In certain embodiments, the pharmaceutical composition is tissue-adhesive. Polymer chains are crosslinked within the tissue and also, they are crosslinked to the ECM.

Penetration of the polymer into the tissue is based on diffusion of polymer chains into cartilage ECM. Polymer length as well as polymer affinity to cartilage ECM influence the degree of penetration. As the polymers of the invention are charged and antifouling, they can penetrate well into cartilage. The polymer will effectively inhibit infiltration of inflammatory cytokines and cells that will initiate tissue degradation.

In case of implants, the coating will inhibit protein accumulation around implant which is the first step of foreign body reaction, and thus will inhibit collagen capsule formation and implant isolation. Also, the coating will inhibit infiltration of immune cells, specifically macrophages to the implant which will otherwise induce tissue degradation.

### Medical treatment

Similarly, within the scope of the present invention is a method of preventing or treating inflammation in a patient in need thereof, comprising administering to the patient a zwitterionic polymer according to the above description.

### Pharmaceutical Compositions, Administration/Dosage Forms and Salts

According to one aspect of the zwitterionic polymer according to the invention, the zwitterionic polymer according to the invention is provided as a pharmaceutical composition, pharmaceutical administration form, or pharmaceutical dosage form.

The skilled person is aware that any specifically mentioned zwitterionic polymer mentioned herein may be present as a pharmaceutically acceptable salt of said polymer. Pharmaceutically acceptable salts comprise the zwitterionic polymer and an oppositely charged counterion. Purely zwitterionic polymers are neutral and not present in form of a salt, as opposed to other charged polymers, but if there is a zwitterionic "copolymer" comprising a charged comonomer, a counterion is present. Non-limiting examples of pharmaceutically acceptable anionic salt forms include acetate, benzoate, besylate, bitatrate, bromide, carbonate, chloride, citrate, edetate, edisylate, embonate, estolate, fumarate, gluceptate, gluconate, hydrobromide, hydrochloride, iodide, lactate, lactobionate, malate, maleate, mandelate, mesylate, methyl bromide, methyl sulfate, mucate, napsylate, nitrate, pamoate, phosphate, diphosphate, salicylate, disalicylate, stearate, succinate, sulfate, tartrate, tosylate, triethiodide and valerate. Non-limiting examples of pharmaceutically acceptable cationic salt forms include aluminium, benzathine, calcium, ethylene diamine, lysine, magnesium, meglumine, potassium, procaine, sodium, tromethamine and zinc.

In certain embodiments of the invention, the zwitterionic polymer of the present invention is typically formulated into pharmaceutical dosage forms to provide an easily controllable dosage of the polymer and to give the patient an elegant and easily handleable product.

Similarly, a dosage form for the prevention or treatment of inflammation is provided, comprising a polymer according to any of the above aspects or embodiments of the invention.

The invention further encompasses a pharmaceutical composition comprising a polymer of the present invention, or a pharmaceutically acceptable salt thereof, and a pharmaceutically acceptable carrier. In further embodiments, the composition comprises at least two pharmaceutically acceptable carriers, such as those described herein.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

### Method of Manufacture and Method of Treatment according to the invention

The invention further encompasses, as an additional aspect, the use of a zwitterionic polymer as identified herein, or its pharmaceutically acceptable salt, as specified in detail above, for use in a method of manufacture of a medicament for the treatment or prevention of inflammation.

Similarly, the invention encompasses methods of treatment of a patient having been diagnosed with a disease associated with inflammation. This method entails administering to the patient an effective amount of the zwitterionic polymer as identified herein, or its pharmaceutically acceptable salt, as specified in detail herein.

The invention further encompasses the following items:
1. A pharmaceutical composition comprising:
- a polymer comprising, or essentially consisting of:
   i. a biocompatible zwitterionic first monomer; and
   ii. a biocompatible crosslinkable second monomer;
   for use in medicine.
2. The pharmaceutical composition for use according to item 1, wherein the first monomer comprises
a. a positively charged moiety;
b. a negatively charged moiety; and
c. a backbone moiety derived from a polymerizable moiety.
3. The pharmaceutical composition for use according to item 2, wherein the polymerizable moiety is selected from acryl, methacryl, vinyl.
4. The pharmaceutical composition for use according to any one of the preceding items, wherein the first monomer is described by a general formula (I) or (II), particularly the first monomer is described by a general formula (I): 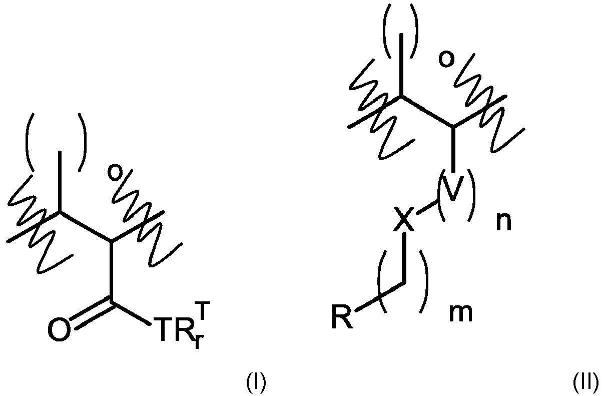 wherein
- T is a heteroatom moiety, particularly T is selected from O, N, NH, more particularly T is NH;
- r is 1 if T is NH or O, and r is 2 if T is N;
- R^{T} is 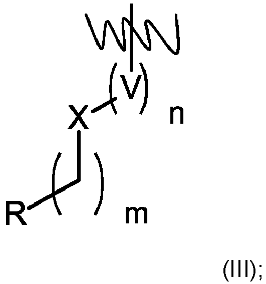
- one of X and R is a positively charged moiety, and the other one is a negatively charged moiety;
- V is selected from the group of -CH₂-, -OCH₂CH₂-, or -CH₂CH₂O-, particularly V is -CH₂-;
- n is selected from 0, 1, 2, 3, 4, 5, 6, 7, 8, 9, 10, particularly n is selected from 1, 2, 3;
- m is selected from 0, 1, 2, 3, 4, particularly m is selected from 0, 1, 2;
- o is selected from 0, 1.

| Moiety | X | R |
|---|---|---|
| positively charged | quaternary ammonium tertiary ammonium secondary ammonium | guanidine quaternary ammonium tertiary ammonium secondary ammonium primary ammonium |
| negatively charged | phosphate thiophosphate | carboxyl sulfate |

5. The pharmaceutical composition for use according to any one of the preceding items, wherein the first monomer comprises a carboxybetaine moiety, a sulfobetaine moiety, a sulfabetaine moiety or a phosphorylcholine moiety, particularly the first monomer is selected from a polymerized form of CBAA (carboxybetaine acrylamide), CBMA (carboxybetaine methacrylate), SBMA (sulfobetaine methacrylate), MPC (2-methacryloyloxyethyl phosphorylcholine), particularly the first monomer is a polymerized form of CBAA.
6. The pharmaceutical composition for use according to any one of the preceding items, wherein the pharmaceutical composition additionally comprises a first crosslinking agent capable of crosslinking said second monomer.
7. The pharmaceutical composition for use according to item 6, wherein the first crosslinking agent is an enzyme.
8. The pharmaceutical composition for use according to item 7, wherein the composition additionally comprises a substrate for said first crosslinking agent.
9. The pharmaceutical composition for use according to item 8, wherein the enzyme is horse-radish peroxidase, and the substrate is H₂O₂.
10. The pharmaceutical composition for use according to item 7, wherein the enzyme is Factor XIII.
11. The pharmaceutical composition for use according to any one of the preceding items 1 to 5, wherein the polymer comprises a crosslinkable moiety, wherein the crosslinkable moiety is crosslinkable induced by heat or induced by light.
12. The pharmaceutical composition for use according to any one of the preceding items, wherein the second monomer is described by a general formula (IV): 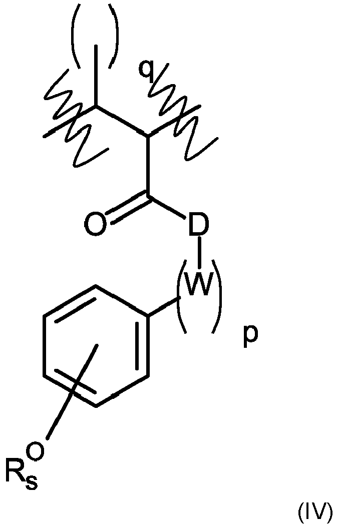 wherein
- p is selected from 0, 1, 2, 3, 4, 5, 6, 7, 8, 9, 10, particularly p is selected from 1, 2, 3;
- q is selected from 0, 1;
- D is a heteroatom moiety, particularly D is selected from O, NH;
- W is selected from the group of -CH₂-, -OCH₂CH₂-, or -CH₂CH₂O-, particularly W is -CH₂-;
- R^{O} is OH;
- s is selected from 1, 2, particularly s is 1.
13. The pharmaceutical composition for use according to any one of the preceding items, wherein the second monomer comprises a crosslinkable moiety selected from tyramine, phenol, catechol, resorcinol.
14. The pharmaceutical composition for use according to any one of the preceding items, wherein the second monomer is selected from a polymerized form of tyramine acrylamide, phenol acrylamide, catechol acrylamide, resorcinol acrylamide, particularly the second monomer is a polymerized form of tyramine acrylamide.
15. The pharmaceutical composition for use according to any one of the preceding items, wherein a ratio of the first monomer to the second monomer is 20:1 to 5:1, particularly the ratio is -10:1.
16. The pharmaceutical composition for use according to any one of the preceding items, wherein the polymer has a molecular mass of 50 kDa to 1000 kDa, particularly of 100 kDa to 500 kDa, more particularly of -200 kDa.
17. A pharmaceutical composition as described in any one of the preceding items 1 to 16 for use in prevention of inflammation of tissue.
18. The pharmaceutical composition for use according to item 17, wherein the pharmaceutical composition is formulated for *in-situ* administration.
19. The pharmaceutical composition for use according to any one of the preceding items 17 to 18, wherein the tissue is an inflamed tissue, or wherein the pharmaceutical composition is for protection of the tissue from inflammation.
20. The pharmaceutical composition for use according to any one of the preceding items 17 to 19, wherein the inflammation of tissue results from an inflammatory disease, particularly from osteoarthritis, or rheumatoid arthritis.
21. The pharmaceutical composition for use according to any one of the preceding items 17 to 20, wherein the tissue is cartilage tissue.
22. A pharmaceutical composition as described in any one of the preceding items 1 to 16 for use in stabilization of a tissue implant.
23. The pharmaceutical composition for use according to item 22, wherein the pharmaceutical composition is administered as a crosslinkable protective penetrant for engineered soft tissues before implantation.
24. The pharmaceutical composition for use according to item 22 or 23, wherein the tissue implant is a hydrogel, a cell-based scaffold or an ECM-like scaffold.
25. The pharmaceutical composition for use according to any one of the preceding items 17 to 24, wherein the pharmaceutical composition is capable of penetrating into tissue, and the pharmaceutical composition is crosslinkable to extra-cellular matrix of cells.

Wherever alternatives for single separable features such as, for example, a first monomer or a second monomer or a medical indication are laid out herein as "embodiments", it is to be understood that such alternatives may be combined freely to form discrete embodiments of the invention disclosed herein. Thus, any of the alternative embodiments for a first monomer may be combined with any of the alternative embodiments of a second monomer and these combinations may be combined with any medical indication mentioned herein.

The invention is further illustrated by the following examples and figures, from which further embodiments and advantages can be drawn. These examples are meant to illustrate the invention but not to limit its scope.

### Description of the Figures

- Fig. 1: Storage modulus of RAFT and FRP.
- Fig. 2: Tissue penetration and in situ gelation. A) Schematic figure of polymer penetration and in situ gelation to tissue explant. B) Fluroscein images of cartilage tissue explant after penetration of fluroscein copolymer and in situ gelation, as well as after 14 days of incubation for both healthy and digested tissue.
- Fig. 3: Native tissue protection. Top) Histological images of bovine articular cartilage explants cultured in healthy (-IL1B) or inflammatory (+IL1B), without and with the protection for 1 week. Bottom) Amount of GAG released to media for each condition as well as conditions with additional Trypsin digestion step to simulate diseased tissue.
- Fig. 4: In vitro engineered tissue protection. Top) Histological images of in vitro produced auricular cartilage discs cultured in healthy (-IL1B) or inflammatory (+IL1B), without and with the protection for 1 week. Bottom) Amount of GAG released to media for each condition.

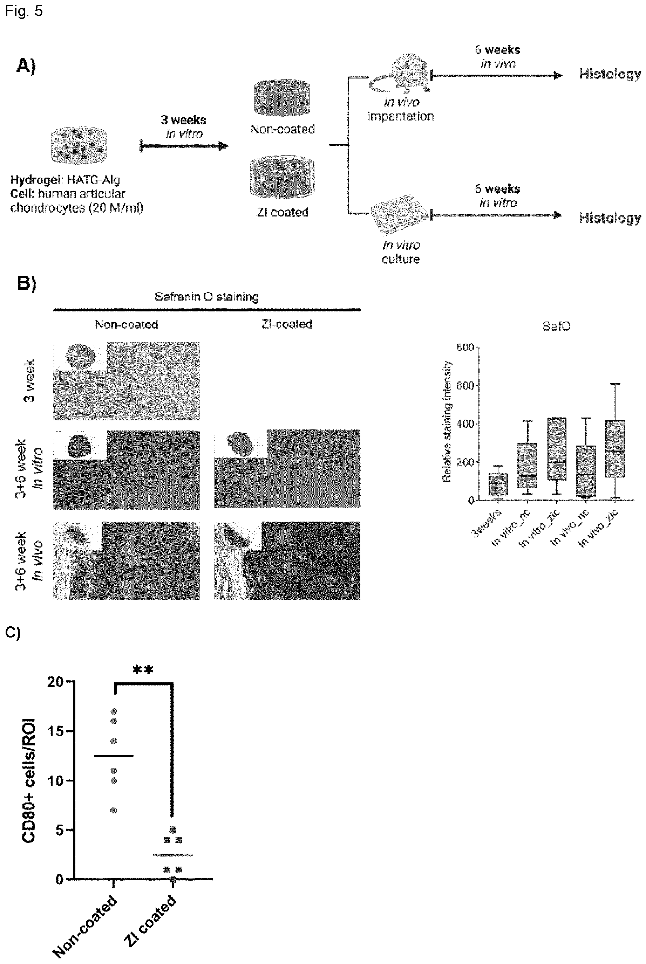

- Fig. 5: In vivo implantation of ZI-coated and non-coated cartilage implants. A) schematic representation of the study. B) Safranin O staining of both In vitro and in vivo samples, as well as quantification of staining intensity, showing preserved GAG content in ZI-coated samples in the in vivo experiment. C) immune cell staining of in vivo samples (CD68: M0 macrophages, CD80: M1 macrophages) showing significantly less macrophage infiltration into ZI-coated samples.
- Fig. 6: Synthesis of polymers and formation of the hydrogel.

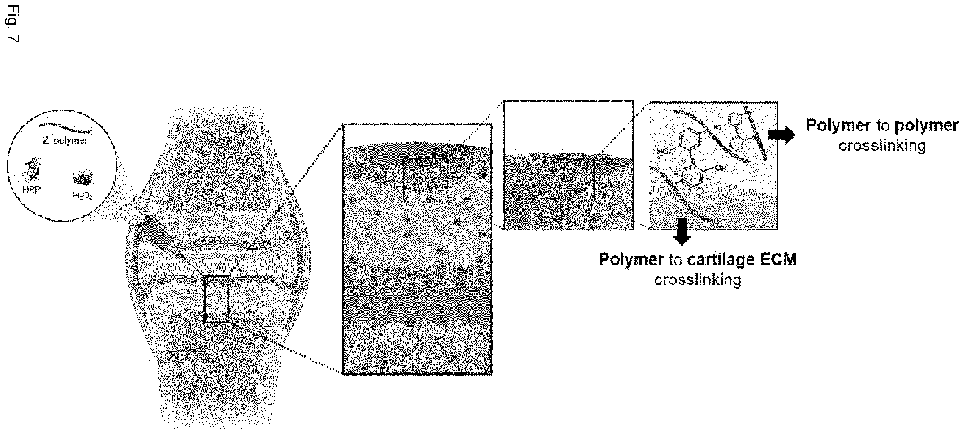

- Fig. 7: *In situ* forming injectable hydrogel shown for knee joint.

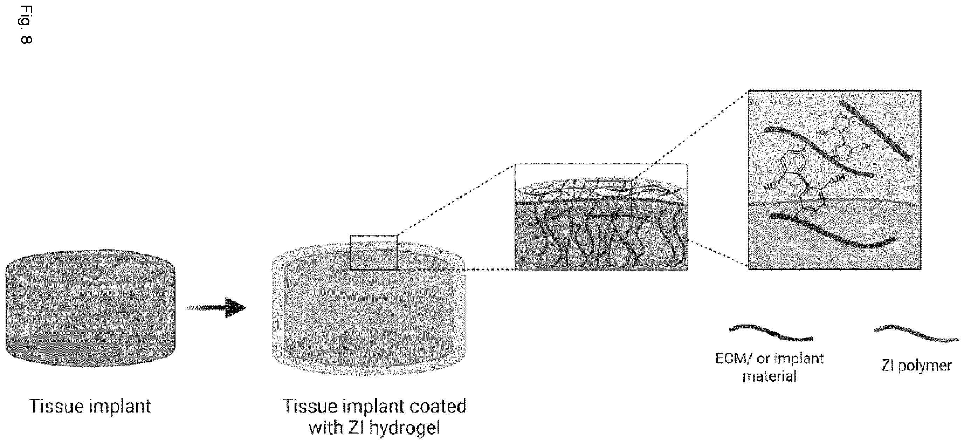

- Fig. 8: Implant coating.

### Examples

### Example 1:

The inventors have developed a protective hydrogel layer that can be easily applied to native or engineered tissues and protect them from inflammation-induced degradation.

The hydrogel layer has tissue penetrative and tissue adhesive properties, increasing its efficiency and performance.

The hydrogel is composed of highly antifouling and biocompatible zwitterionic polymers, that provide both tissue penetration as well as an efficient barrier inhibiting and repelling inflammatory cells and cytokines from attacking the tissue.

The zwitterionic polymers are crosslinked with a tissue-adhesive crosslinking method that is highly biocompatible and has tissue adhesiveness as well as in vivo stability.

The protective hydrogel can be applied either in situ as injectable formulation, or in vitro as a protective coating for engineered soft tissues before implantation.

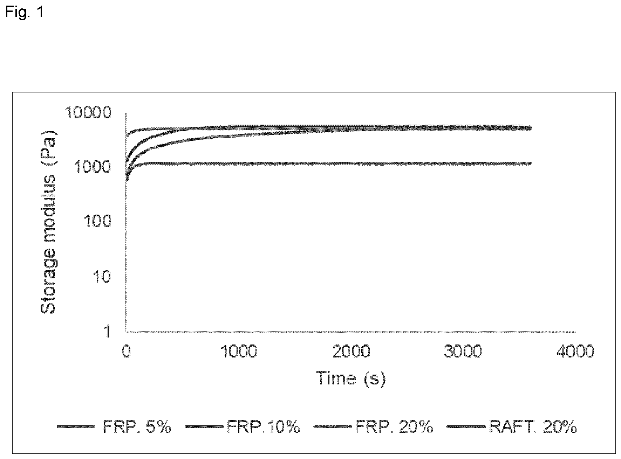

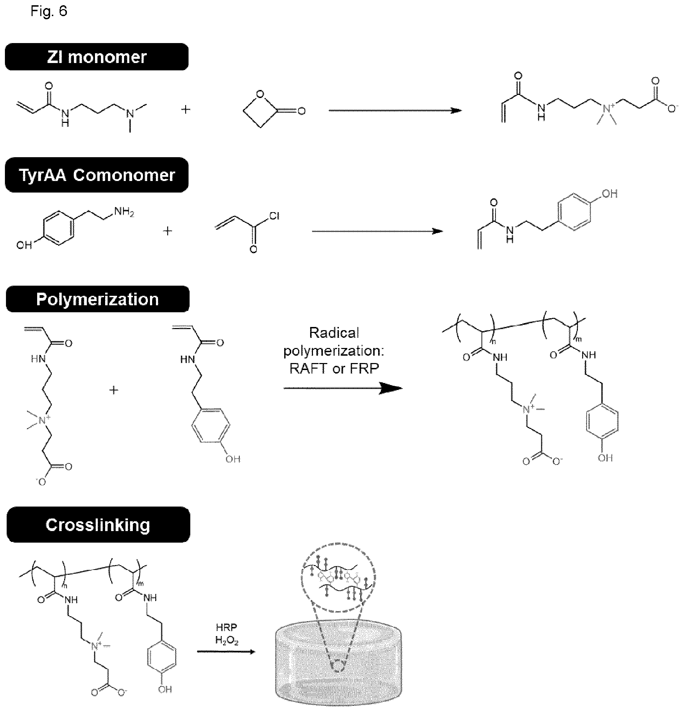

As an example, the inventors have synthesized zwitterionic copolymers via RAFT and FRP polymerization, composed of carboxybetaine acrylamide (CBAA) as the zwitterionic monomer and a novel tyramine acrylamide (TyrAA) comonomer as a functional group to provide enzymatic crosslinking of polymer chains in presence of horseradish peroxide (HRP) enzyme and hydrogen peroxide. The inventors have optimized polymerization as well as crosslinking protocols and have shown effective hydrogel crosslinking (Figure 1 and 6).

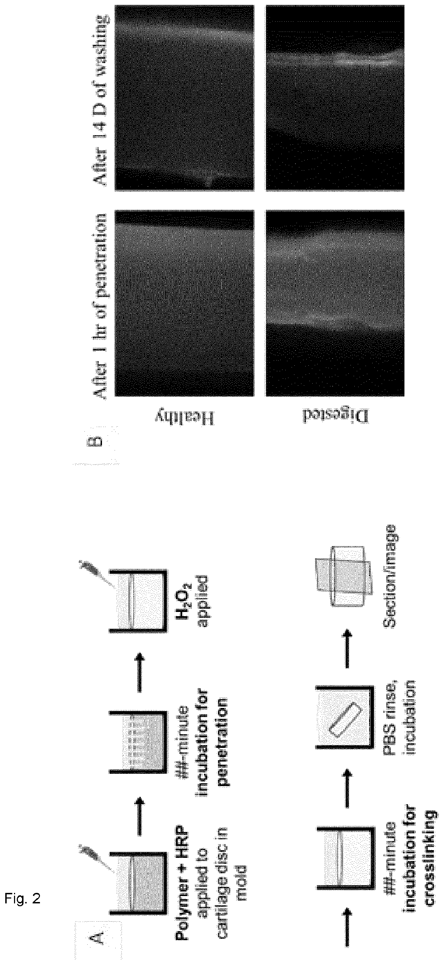

The inventors have shown effective polymer penetration and in situ gelation of the hydrogel into bovine cartilage explants as a model tissue, as well as its long-term retention into the tissue (Figure 2)

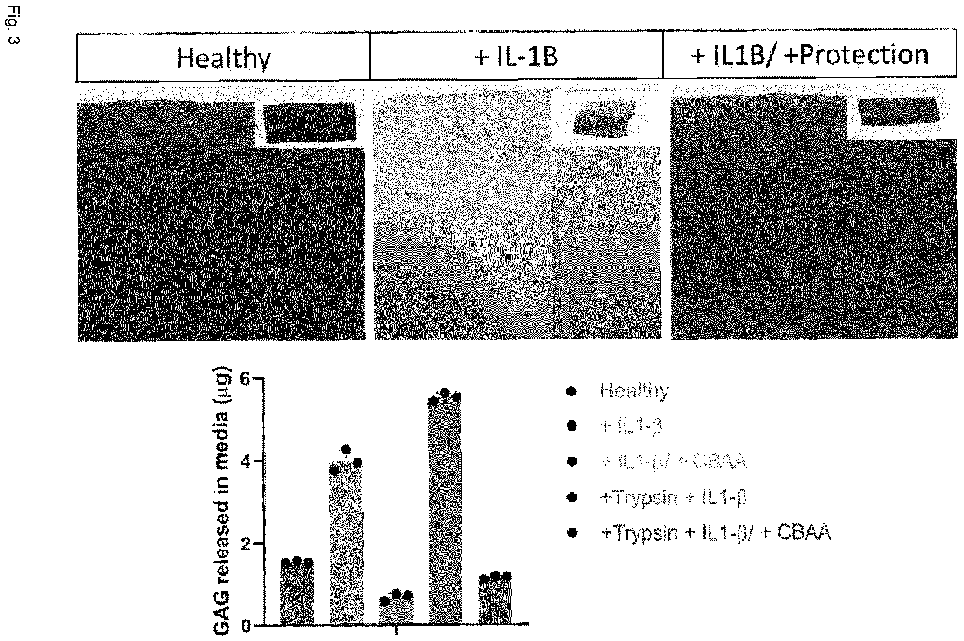

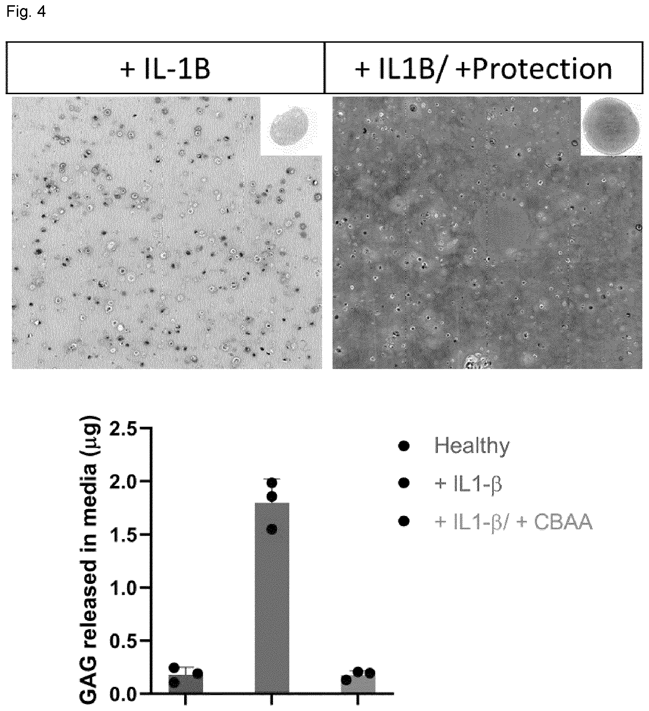

Also, the inventors have shown protective effect of the hydrogel on both native tissue explants (Figure 3) and in vitro engineered tissues (Figure 4) cultured in inflammatory media containing 10 ng/ml IL1B, as the most effective inflammatory cytokine inducing tissue degradation.

As the native tissue model, the inventors have used bovine articular cartilage explants, and cultured them in the aforementioned inflammatory media. The inventors have seen a striking difference between samples that are protected via the protective hydrogel layer and the ones that are not protected, in terms of lost tissue integrity and content visualized by histological images. Moreover, the inventors have analyzed the supernatant culture media for the amount of released extracellular matrix content, which is mainly glycosaminoglycans (GAG) in terms of cartilage tissue, and have observed that the amount of released tissue to media is significantly lower in case of protected tissue (Figure 3).

As the implant tissue model, the inventors used in vitro produced auricular tissue as a model tissue and cultured them in the inflammatory media with and without our protective hydrogel layer. The inventors could again see the effect of protective layer both in tissue integrity visualized via histology and in amount of released tissue to the media (Figure 4).

### NMR data

### CBAA:

1 H NMR (300 MHz, ) δ 6.25 - 6.06 (m), 5.69 (ddd, *J* = 7.2, 1.5, 0.6 Hz), 4.70, 3.51 - 3.44 (m), 3.32 - 3.21 (m), 3.14 - 2.99 (m), 2.99, 2.56 (ddt, *J* = 6.7, 5.8, 1.0 Hz), 2.02 - 1.85 (m).

### TyrAA:

1 H NMR (300 MHz, ) δ 7.06 - 7.01 (m), 6.75 - 6.70 (m), 6.05 (d, *J* = 0.4 Hz), 6.03, 6.01 (dd, *J* = 1.4, 0.4 Hz), 5.60 (dd, *J* = 1.5, 0.4 Hz), 5.57 (dd, *J* = 1.4, 0.4 Hz), 4.70, 3.38, 3.36, 3.34, 2.67, 2.65, 2.64.

### Polymer-FRP:

1 H NMR (300 MHz, ) δ 7.81, 4.70 (d, *J* = 0.3 Hz), 3.87 (qd, *J* = 6.9, 0.4 Hz), 3.46, 3.33 - 2.84 (m), 2.76, 2.74 (t, *J* = 0.5 Hz), 2.55 (d, *J* = 6.5 Hz), 1.88.

### Polymer-RAFT:

1 H NMR (300 MHz, ) δ 7.81, 4.82 - 4.59 (m), 4.20 - 3.72 (m), 3.12, 3.58 - 2.39 (m), 2.22 - 1.71 (m), 1.44.

## Claims

1. A pharmaceutical composition for use in medicine, said composition comprising:
- a polymer comprising, or essentially consisting of:
i. a biocompatible zwitterionic first monomer; and
ii. a biocompatible crosslinkable second monomer;
wherein the first monomer comprises
a. a positively charged moiety;
b. a negatively charged moiety; and
c. a backbone moiety derived from a polymerizable moiety.

2. The pharmaceutical composition for use according to claim 1, wherein the first monomer is described by a general formula (I) or (II): 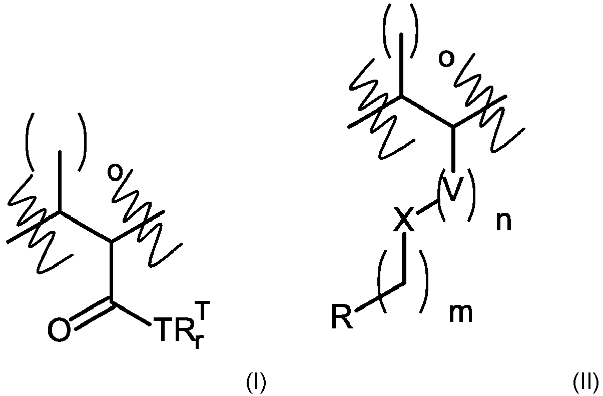 wherein
- T is selected from the group comprising O, N, NH;
- R^{T} is 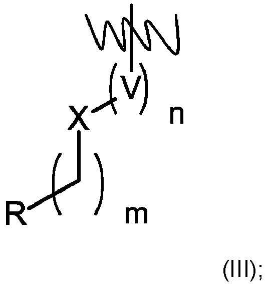
- r is 1 if T is NH or O, and r is 2 if T is N;
- one of X and R is a positively charged moiety, and the other one is a negatively charged moiety, X and R being selected from the following table:
| Moiety | X | R |
|---|---|---|
| positively charged | quaternary ammonium tertiary ammonium secondary ammonium | guanidine quaternary ammonium tertiary ammonium secondary ammonium primary ammonium |
| negatively charged | phosphate thiophosphate | carboxyl sulfate |
- V is selected from the group comprising -CH₂-, -OCH₂CH₂-, or -CH₂CH₂O-;
- n is selected from the group comprising 0, 1, 2, 3, 4, 5, 6, 7, 8, 9, 10;
- m is selected from the group comprising 0, 1, 2, 3, 4;
- o is selected from 0 and 1.

3. The pharmaceutical composition for use according to any one of the preceding claims, wherein the first monomer is selected from the group comprising a polymerized form of CBAA (carboxybetaine acrylamide), a polymerized form of CBMA (carboxybetaine methacrylate), a polymerized form of SBMA (sulfobetaine methacrylate), a polymerized form of MPC (2-methacryloyloxyethyl phosphorylcholine).

4. The pharmaceutical composition for use according to any one of the preceding claims, wherein the pharmaceutical composition
i. comprises an enzyme capable of crosslinking said second monomer, or
ii. crosslinking of the crosslinkable moiety can be induced by heat or induced by light.

5. The pharmaceutical composition for use according to claim 4, wherein
a. the enzyme is horse-radish peroxidase, and the composition additionally comprises is H₂O₂, as a substrate or
b. the enzyme is Factor XIII.

6. The pharmaceutical composition for use according to any one of the preceding claims, wherein the second monomer is described by a general formula (IV): 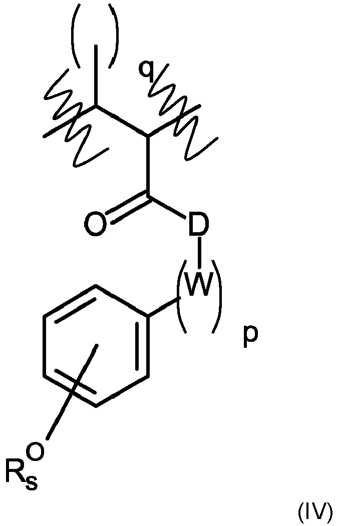 wherein
- p is selected from the group comprising 0, 1, 2, 3, 4, 5, 6, 7, 8, 9, 10;
- q is selected from 0, 1;
- D is selected from O, NH;
- W is selected from the group comprising -CH₂-, -OCH₂CH₂-, or -CH₂CH₂O-;
- R^{O} is OH;
- s is selected from 1, 2.

7. The pharmaceutical composition for use according to any one of the preceding claims, wherein the second monomer comprises a crosslinkable moiety selected from the group comprising tyramine, phenol, catechol, resorcinol.

8. The pharmaceutical composition for use according to claim 7, wherein the second monomer is selected from the group comprising a polymerized form of tyramine acrylamide, a polymerized form of phenol acrylamide, a polymerized form of catechol acrylamide, a polymerized form of resorcinol acrylamide.

9. The pharmaceutical composition for use according to any one of the preceding claims, wherein
a ratio of the first monomer to the second monomer is 20:1 to 5:1, and/or
- the polymer has a molecular mass of 50 kDa to 1000 kDa.

10. A pharmaceutical composition as described in any one of the preceding claims 1 to 9 for use in prevention or treatment of tissue inflammation.

11. The pharmaceutical composition for use according to claim 10, wherein the pharmaceutical composition is formulated for *in-situ* administration.

12. The pharmaceutical composition for use according to any one of the preceding claims 10 to 11, wherein the tissue inflammation results from, or occurs in association with, osteoarthritis, or rheumatoid arthritis.

13. The pharmaceutical composition for use according to any one of the preceding claims 10 to 12, wherein the tissue is cartilage tissue.

14. A pharmaceutical composition as described in any one of the preceding claims 1 to 9 for use in medicine by administration as a coating to a tissue implant.

15. The pharmaceutical composition for use according to claim 14, wherein the tissue implant is a hydrogel, a cell-based scaffold or an ECM-like scaffold.
